# EUROPEAN PATENT APPLICATION

(11) **EP 1 947 458 A1**
(43) Date of publication of application: **23.07.2008**
(21) Application number: 07000858.6
(22) Date of filing: 17.01.2007
(51) Int. Cl.: G01N 33/543, G01N 33/558, G01N 33/52

(54) **Using colour conjugated chitosan and chitosan oligo-saccharides in the manufacture of a lateral-flow test device**

(71) Applicant: The Jordanian Pharmaceutical Manufacturing Co. Ltd., 11710 Naor (JO)
(72) Inventor: Badwan, Adnan, Dr., 11185 Amman (JO); Abdel-Quader Murshed Mohammed, Murshed, 11118 Amman (JO)
(74) Representative: Winkler, Andreas Fritz Ernst

(57) **Abstract**

The present invention refers to a lateral-flow immunochromatographic test device comprising an optionally dye conjugated water-soluble chitosan and a dye conjugated water-soluble chitosan oligosaccharide. The present invention further refers to a use of said test device for observing a sample fluid flow. The present invention also refers to a use of an optionally dye conjugated water-soluble chitosan solution as a blocking solution, and to a dye conjugated chitosan oligosaccharide solution as a masking solution in the manufacture of said test device.

## Description

The present invention refers to a lateral-flow immunochromatographic test device comprising an optionally dye conjugated water-soluble chitosan and a dye conjugated water-soluble chitosan oligosaccharide. The present invention further refers to a use of said test device for observing a sample fluid flow. The present invention also refers to a use of an optionally dye conjugated water-soluble chitosan solution as a blocking solution, and to a dye conjugated chitosan oligosaccharide solution as a masking solution in the manufacture of said test device.

### Background of the Invention

In recent years, the *in vitro* diagnostics (IVD) industry has made enormous efforts to develop immunochromatographic tests. Such tests have found applications in both clinical and non-clinical fields. A clinical utility of this test format has been shown for more than 150 different analytes, and many of them are target now of commercially available diagnostic products.

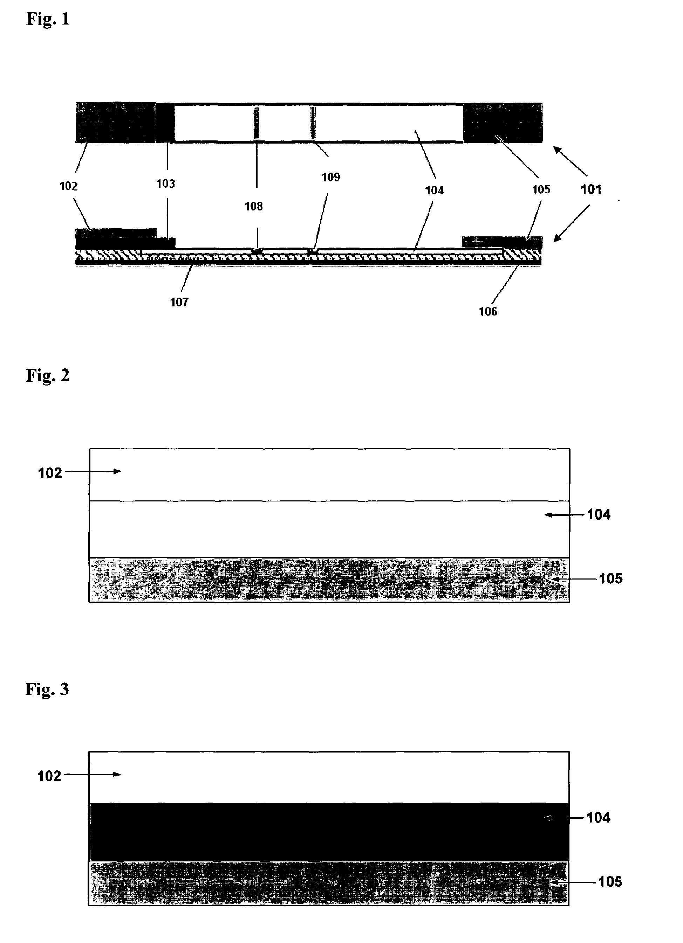

Rapid immunochromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

It is an object of the present invention to provide a lateral-flow test device having advantageous features.

### Detailed Description of the Invention

The object of the present invention is solved by a lateral-flow immunochromatographic test device comprising a water-soluble chitosan and a dye conjugated water-soluble chitosan oligosaccharide.

In one embodiment, the test device further comprises a negatively charged membrane, preferably a nitrocellulose membrane.

In one embodiment, the test device further comprises at least one protein printed onto the membrane.

In one embodiment, the chitosan is a dye conjugated water-soluble chitosan.

In one embodiment, the colour provided by the dye conjugated chitosan is different from the colour provided by the dye conjugated chitosan oligosaccharide.

In one embodiment, the dye conjugated to chitosan oligosaccharide is selected from acid red 88, acid red 27, and acid blue 113.

In one embodiment, the dye conjugated to chitosan is selected from acid red 88, acid red 27, and acid blue 113.

In a particularly preferred embodiment, the dye conjugated to chitosan is acid blue 113, and the dye conjugated to chitosan oligosaccharide is acid red 88 or acid red 27.

In one embodiment, the test device further comprises at least one coloured latex particle labelled antibody or antigen.

The object of the present invention is further solved by a use of a test device according to the present invention for observing a sample fluid flow.

The object of the present invention is further solved by a use of a water-soluble chitosan solution as a blocking solution in the manufacture of a test device according to the present invention.

In one embodiment, the chitosan is a dye conjugated water-soluble chitosan.

In one embodiment, the dye conjugated to chitosan is selected from acid red 88, acid red 27, and acid blue 113.

The object of the present invention is further solved by a use of a dye conjugated water-soluble chitosan oligosaccharide solution as a masking solution in the manufacture of a test device according the present invention.

In one embodiment, the dye conjugated to chitosan oligosaccharide is selected from acid red 88, acid red 27, and acid blue 113.

The object of the present invention is further solved by a use of a dye conjugated water-soluble chitosan solution for quality control in the manufacture of a lateral-flow immunochromatographic test device.

In one embodiment, the test device comprises a negatively charged membrane, preferably a nitrocellulose membrane.

In one embodiment, the test device further comprises at least one protein printed onto the membrane.

In one embodiment, the dye conjugated to chitosan is selected from acid red 88, acid red 27, and acid blue 113.

Thus, the present invention provides a system comprising a chitosan blocking solution and a chitosan oligosaccharide masking solution. An ordinary chitosan solution has no colour while an ordinary chitosan oligosaccharide solution has a yellowish colour. Both, chitosan and chitosan oligosaccharides can be coloured by conjugation with acid red and/or acid blue dyes. In the system of the present invention, the chitosan solution can be colourless or can be coloured by dye conjugated chitosan. The chitosan oligosaccharide solution is coloured by dye conjugated chitosan oligosaccharide. The coloured chitosan solutions can be employed to produce different coloured nitrocellulose membranes that will make a new feature of rapid immunochromatographic test devices.

If the chitsoan solution is colourless, the dye conjugated chitosan oligosaccharide solution will colour the white membrane. The mask provided by chitosan oligosaccharide is removed with the sample flow, thus supporting the observation of the sample fluid flow. If the chitosan solution is coloured, e.g. blue, the dye conjugated to chitosan oligosaccharide provides a different colour, e.g. red.

The advantage of the invention is that it becomes possible to control the colour of the test and sample lines by varying the contrast between the coloured lines and the membrane background. This can be further supported by the use of coloured latex particle labelled antibodies or antigens instead of colloidal gold labelled proteins. On this basis, the detection limit of an analyte measured by a test device of the present invention can be improved.

### Detailed Description of the Invention

### Brief Description of the Figures

- Figure 1: shows top and side views of a typical rapid-flow immunochromatographic test device in the form of a test strip 101 including a sample pad 102, a conjugate pad 103, a membrane 104 that incorporates capture reagents, an absorbent pad 105, an adhesive 106, a supporting backing 107, a test zone 108, and a control zone 109.
- Figure 2: shows a top view of a printed nitrocellulose membrane during the manufacturing of a rapid-flow immunochromatographic test device.
- Figure 3:: shows a top view of a printed nitrocellulose membrane after the application of chitosan during the manufacturing of a rapid-flow immunochromatographic test device.
- Figure 4:: shows top and side views of a rapid-flow immunochromatographic test device treated with chitosan, e.g. before sample application.
- Figure 5:: shows top and side views of a rapid-flow immunochromatographic test device treated with a blue colour conjugated water-soluble chitosan solution and a red colour conjugated chitosan oligosaccharide solution after sample application during sample flow.

### EXAMPLES

### EXAMPLE 1: Use of colour conjugated chitosan and chitosan oligosaccharide solution for quality control

The use of blue colour conjugated water-soluble chitosan solution allows for the quality control of printing proteins onto nitrocellulose membranes.

A nitrocellulose membrane is treated with a blue colour conjugated water-soluble chitosan solution. The blue colour is due to a dye such as acid blue 113 (CI26360, also referred to as AB113). Chitosan that contains amino groups as active groups blocks all active nitro groups on the surface of the nitrocellulose membrane except for the protein-printed areas (e.g. lines). If a blue colour conjugated water-soluble chitosan solution is used, cuts within the protein-printed areas can be easily detected. Without application of the blue colour conjugated water-soluble chitosan solution, printed protein areas can not be visually distinguished.

### EXAMPLE 2: Use of colour conjugated chitosan and chitosan oligosaccharide solution for sample fluid flow observation

The use of blue colour conjugated water-soluble chitosan solution and red colour conjugated chitosan oligosaccharide solution allows for the observation of sample fluid flow.

In a first step, a nitrocellulose membrane is treated with a blue colour conjugated water-soluble chitosan solution. The blue colour is due to a dye such as acid blue 113 (CI 26360, also referred to as AB113). Chitosan that contains amino groups as active groups blocks all active nitro groups on the surface of the nitrocellulose membrane except for the protein-printed areas (e.g. lines).

In a second step, the membrane is treated with a red colour conjugated water soluble chitosan oligosaccharide solution. The red colour is due to a dye such as acid red 88 (CI 15620, also referred to as AR88) or acid red 27 (CI 16185, also referred to as AR27).

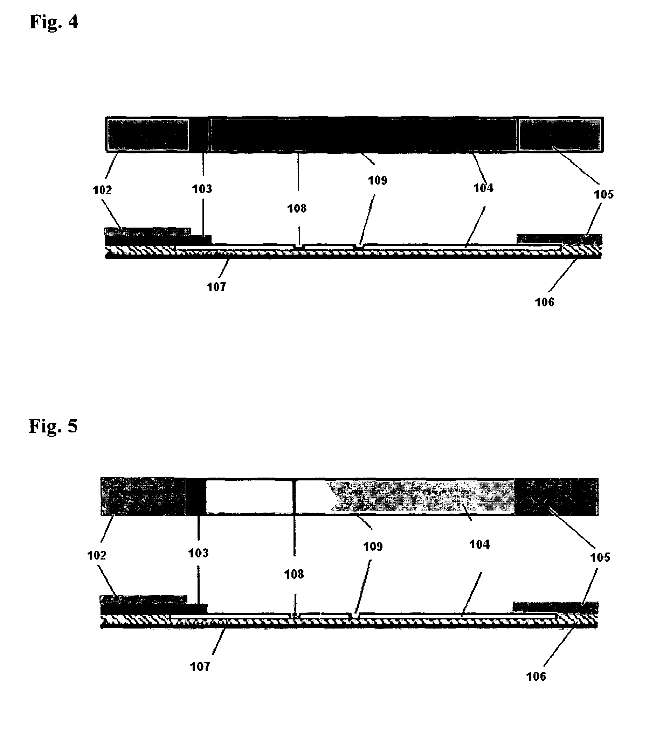

After application of a sample onto the sample pad, the sample fluid passes through the nitrocellulose membrane toward the absorbent pad (Fig. 5), and with its flow it washes the nitrocellulose membrane and removes the red colour conjugated chitosan oligosaccharide. As a result of the interaction between the analyte (bound to the conjugated) within the sample and the printed protein (e.g. antibodies or antigens) a red coloured line appears on the blue coloured background.

### EXAMPLE 3: Use of colourless chitosan and colour conjugated chitosan oligosaccharide solution for sample fluid flow observation

The use of colourless water-soluble chitosan solution and red colour conjugated chitosan oligosaccharide solution allows for the observation of sample fluid flow.

In a first step, a nitrocellulose membrane is treated with a colourless water-soluble chitosan solution. Chitosan that contains amino groups as active groups blocks all active nitro groups on the surface of the nitrocellulose membrane except for the protein-printed areas (e.g. lines).

In a second step, the membrane is treated with a red colour conjugated water soluble chitosan oligosaccharide solution. The red colour is due to a dye such as acid red 88 (CI 15620, also referred to as AR88) or acid red 27 (CI 16185, also referred to as AR27).

After application of a fluid sample onto the sample pad, the sample fluid passes through the nitrocellulose membrane toward the absorbent pad (Fig. 5), and with its flow it washes the nitrocellulose membrane and removes the red colour conjugated chitosan oligosaccharide. As a result of the interaction between the analyte (bound to the conjugated) within the sample an the printed protein (e.g. antibodies or antigens) a red coloured line appears on the colourless background.

## Claims

1. A lateral-flow immunochromatographic test device comprising a water-soluble chitosan and a dye conjugated water-soluble chitosan oligosaccharide.

2. The test device according to claim 1, further comprising a negatively charged membrane, preferably a nitrocellulose membrane.

3. The test device according to claim 1 or 2, further comprising at least one protein printed onto the membrane.

4. The test device according to any of claims 1 to 3, wherein the chitosan is a dye conjugated water-soluble chitosan.

5. The test device according to claim 4, wherein the colour provided by the dye conjugated chitosan is different from the colour provided by the dye conjugated chitosan oligosaccharide.

6. The test device according to any of claims 1 to 5, wherein the dye conjugated to chitosan oligosaccharide is selected from acid red 88, acid red 27, and acid blue 113.

7. The test device according to claim 4 or 5, wherein the dye conjugated to chitosan is selected from acid red 88, acid red 27, and acid blue 113.

8. The test device according to any of claims 4 to 7, wherein the dye conjugated to chitosan is acid blue 113, and the dye conjugated to chitosan oligosaccharide is acid red 88 or acid red 27.

9. The test device according to any of claims 1 to 8, further comprising at least one coloured latex particle labelled antibody or antigen.

10. A use of a test device according to any of claims 1 to 9 for observing a sample fluid flow.

11. A use of a water-soluble chitosan solution as a blocking solution in the manufacture of a test device according to any of claims 1 to 9.

12. The use according to claim 11, wherein the chitosan is a dye conjugated water-soluble chitosan.

13. The use according to claim 12, wherein the dye conjugated to chitosan is selected from acid red 88, acid red 27, and acid blue 113.

14. A use of a dye conjugated water-soluble chitosan oligosaccharide solution as a masking solution in the manufacture of a test device according to any of claims 1 to 9.

15. The use according to claim 14, wherein the dye conjugated to chitosan oligosaccharide is selected from acid red 88, acid red 27, and acid blue 113.

16. Use of a dye conjugated water-soluble chitosan solution for quality control in the manufacture of a lateral-flow immunochromatographic test device.

17. The use according to claim 16, wherein the test device comprises a negatively charged membrane, preferably a nitrocellulose membrane.

18. The use according to claim 17, wherein the test device further comprises at least one protein printed onto the membrane.

19. The use according to claim 18, wherein the dye conjugated to chitosan is selected from acid red 88, acid red 27, and acid blue 113.
